# EUROPÄISCHE PATENTANMELDUNG

(11) **EP 0 672 673 A1**
(43) Veröffentlichungstag der Anmeldung: **20.09.1995**
(21) Anmeldenummer: 95103404.0
(22) Anmeldetag: 09.03.1995
(51) Int. Cl.: C07F 9/38, A61K 51/04, C07F 9/40

(54) **Nichtcyclische Chelatbildner auf Basis von Aminodialkylphosphoroxiden zur Herstellung von Technetium- oder Rheniumkomplexen**

(30) Priorität: 15.03.1994 DE 4408729
(71) Anmelder: HOECHST AKTIENGESELLSCHAFT, D-65929 Frankfurt am Main (DE)
(72) Erfinder: Stahl, Wilhelm, Dr., D-65510 Idstein (DE); Walch, Axel, Dr., D-60487 Frankfurt am Main (DE); Doll, Wilfried, D-64569 Nauheim (DE); Kuhlmann, Ludwig, Dr., D-65439 Flörsheim (DE); Pütter, Dietrich, D-65933 Frankfurt am Main (DE)

(57) **Zusammenfassung**

Die Erfindung betrifft nichtcyclische Chelatbildner auf Basis von Aminodialkylphosphoroxiden der Formel (I)

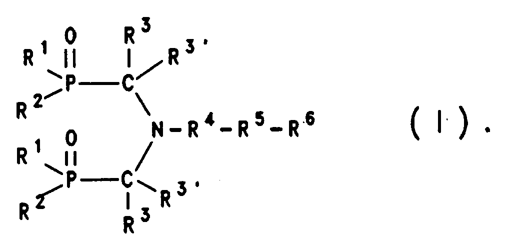

Die erfindungsgemäßen Verbindungen werden verwendet zur Markierung von Substanzen, insbesondere mit radioaktiven Technetium- oder Rheniumisotopen. Ebenso dienen diese Verbindungen als Diagnostikum oder Therapeutikum, und in Form eines Testkits zum Nachweis von Tumoren.

## Beschreibung

Die Erfindung betrifft nichtcyclische Chelatbildner auf Basis von Aminodialkylphosphoroxiden, ihre Verwendung zur Markierung von Substanzen mit insbesondere radioaktiven Technetium- oder Rheniumisotopen, sowie die Verwendung der Chelate in diagnostischen und therapeutischen Verfahren.

In den vergangenen Jahren wuchs das Verlangen, chemische Verbindungen mit radioaktiven Nukliden zu markieren. Vor allem im Bereich der medizinischen Diagnostik, wo pathologische Zustände bereits durch Substanzen angezeigt werden können, die im Organismus nur in ppm oder gar in noch geringeren Konzentrationen vorkommen, kann man heute nicht mehr auf radioaktiv markierte Substanzen verzichten.

Insbesondere Technetium-99m ist wegen seiner günstigen physikalischen Eigenschaften (Fehlen einer Korpuskularstrahlung, γ-Energie von 140 keV und Halbwertszeit von 6 Stunden) und der damit verbundenen geringen Strahlenbelastung zum wichtigsten Radionuklid in der nuklearmedizinischen Diagnostik geworden.

Technetium-99m, das sich aus Nuklidgeneratoren gewinnen läßt, liegt zunächst als Pertechnetat vor, das in dieser Form, z. B. für die Schilddrüsen- und Hirnszintigraphie geeignet ist. Die Szintigraphie anderer Organe mittels Technetium-99m gelingt mit Hilfe bestimmter "Transportsubstanzen", die einerseits in der Lage sind, Technetium zu binden und andererseits das Radionuklid im Zielorgan mit hoher Selektivität anzureichern. Zur Markierung der organspezifischen Transportsubstanz mit Technetium-99m muß das aus dem Nuklidgenerator eluierte Pertechnetat zuerst in eine niedrigere Oxidationsstufe übergeführt werden. In dieser reduzierten Form bildet Technetium mit der organspezifischen Substanz mehr oder weniger stabile Verbindungen. Für die Knochenszintigraphie werden z. B. Tc-99m-Phosphorsäure-Derivate, vor allem organische Phosphonsäuren, eingesetzt. So liegt in der im Europäischen Patent 002485 beschriebenen Markierungseinheit das Natriumsalz der 3,3-Diphosphon-1,2-propandicarbonsäure als organspezifische Transportsubstanz vor. In dem Europäischen Patent 108253 werden Tc-99m-Tri- und Tetraphosphonsäuren zur szintigraphischen Darstellung des Retikulo-endothelialen Systems (RES), insbesondere der Leber, beschrieben. Der Tc-99m-Komplex mit Diethylentriaminpentaessigsäure (DTPA) findet zur Diagnostik von Nierenerkrankungen bzw. pathologischen Hirnprozessen Anwendung.

Zur Markierung bestimmter Substanzen mit Technetium-99m und zur Herstellung von für den klinischen Routinebedarf geeigneten Testkits wurden spezielle Verfahren entwickelt und beschrieben. Zur Präparierung von Markierungsbestecken für biologisch interessante Makromoleküle, insbesondere Porphyrine, Dextrane, Cytochrome und Myoglobin, wird eine Methode beschrieben (G. D. Zanelli, D. Ellison, M. P. Barrowcliffe, Nucl. Med. Commun. 8, 199 bis 206, 1987), bei der die zu markierende Substanz zusammen mit p-Aminobenzoesäure und einer salzsauren SnCl₂-Lösung lyophilisiert wird. Zur Rekonstitution und Markierung dieses Kits gibt man Tc-99m-Generatoreluat, das vorher mit genügend Pufferlösung, z. B. Citrat-Natriumchlorid-Puffer pH 9,5, verdünnt wurde, hinzu. Für säureempfindliche Substanzen ist diese Methode jedoch nicht geeignet.

Bei einem anderen Verfahren (E. K. J. Pauweis, R. I. J. Feitsma, internationale Patentanmeldung WO 86/03010) wird durch vierstündiges Erhitzen in stark salzsaurer Lösung auf 140°C Tc-99m-Pertechnetat zuerst reduziert und an einer Verbindung, die eine Aminogruppe enthält, z. B. Dimethylformamid, gebunden. Das reaktionsfähige Tc-99m-markierte Zwischenprodukt, das als schwerlösliche kristalline Substanz ausfällt, wird in einer Pufferlösung, z. B. Natriumcarbonatlösung, durch einstündige Inkubation bei Raumtemperatur mit der zu markierenden Verbindung umgesetzt. Die Methode arbeitet zwar zinnfrei, ist aber wegen der aufwendigen Verfahrensschritte für die Routineanwendung kaum geeignet.

Zur Markierung von Proteinen, insbesondere Antikörpern, kennt man zwei verschiedene Wege. Bei der direkten Methode wird das reduzierte Technetium-99m durch Donorgruppen (Amino-, Amid-, Thiol- etc.) des Proteins gebunden.

Solche Verfahren sind in dem Europäischen Patent 005638 und dem US-Patent 4.478.815 beschrieben. Dort werden Zinn-II-Salze im Überschuß zur gleichzeitigen reduktiven Spaltung von Disulfid-Brücken und zur Reduktion des zugesetzten Tc-99m-Pertechnetats benutzt. Im allgemeinen benötigt man zur Spaltung der -S-S-Bindung längere Inkubationszeiten (24 Stunden), wobei F(ab')₂-Fragmente partiell zu Fab'-Fragmenten gespalten werden. Neuere Literaturangaben (z. B. Journal of Nuclear Medicine 27 (1986), Seiten 685 bis 693 und 1315 bis 1320 sowie International Journal of Nuclear Medicine Biology 12 (1985) Seiten 3 bis 8) zeigen, daß das Verhältnis der beiden Fragmente abhängig ist von der "Bezinnungsreaktion" und daß sich das Verhältnis der beiden Komponenten nach der Tc-99m-Markierung nicht mehr nennenswert ändert, wobei die Hauptkomponente Tc-99m-markiertes F(ab') ist. In allen Fällen mußte das markierte F(ab')-Fragment nachgereinigt werden, da trotz mindestens 30-minütiger Reaktionszeit keine quantitative Umsetzung des Pertechnetats erreicht wurde.

Bei einer schnellen chemischen Methode zur Tc-99m-Markierung humaner Plasmaproteine (D. W. Wong, F. Mishkin, T. Lee, J. Nucl. Med. 20, 967 bis 972, 1979) wird Pertechnetat zuerst in saurer Lösung durch Zinn-II-Ionen reduziert und das reduzierte Technetium dann mit dem Protein umgesetzt.

Unter Zuhilfenahme von bifunktionalen Komplexbildnern läßt sich eine stabile Markierung von Substanzen mit Radioisotopen erreichen. In dem US-Patent 4.479.930 werden die cyclischen Anhydride von DTPA und EDTA als Chelatisierungsmittel nicht nur für In-111 und Ga-67, sondern auch für Tc-99m angeführt. In dem europäischen Patent 35765 wird die Verwendung von Deferoxamin als Komplexierungsagens für Technetium-99m an Proteine erwähnt. In der internationalen Patentanmeldung WO 85/3063 werden die partiell reduzierten Disulfid-Brücken im Antikörper mit dem Natriumsalz des Tetrachlornitridotechnetats, das durch Reaktion von Pertechnetat mit Natriumazid vorher präpariert werden muß, umgesetzt. In der europäischen Patentanmeldung 194853 benutzt man ebenfalls durch Reduktion in Antikörperfragmenten erzeugte freie Thiolgruppen zur Bindung von [(7-Maleimidoheptyl)imino-bis(ethylennitrilo)]-tetraessigsäure als Chelatkomplex. Die Kupplung des Komplexes an den Antikörper erfolgt über die Reaktion der SH-Gruppen mit der Doppelbindung im Maleinimidteil der Komplexverbindung, während das radioaktive Metallion über die Nitrilodiessigsäure-Reste komplexiert wird.

Metallothionein, ein metallbindendes Protein mit einem Molekulargewicht von 6000 und einem hohen Cysteinanteil im Molekül wurde als Komplexbildner in Antikörper eingeführt (G. L. Toman, R. J. Hadjian, M. M. Morelock et al, J. Nucl. Med. 25, 20, 1984). Durch Austausch mit Tc-99m-Glucoheptonat ließ sich das Antikörper-Metallothionein-Konjugat mit Technetium markieren. Der Austausch blieb jedoch unvollständig, so daß eine Nachreinigung erforderlich war. Mehrere Bisthiosemicarbazon-Liganden wurden ebenfalls als bifunktionelle Chelatbildner beschrieben (Y. Arano, A. Yokoyama, H. Magat et al., Int. J. Nucl. Med. Biol. 12, 425 bis 430, 1986). p-Carboxyethylphenylglyoxal-di(N-methylthiosemicarbazon) wurde mit humanem Serumalbumin konjugiert. Der Tc-99m-markierte 1 : 1-Komplex zeigte eine gewisse Instabilität, während Komplexe mit einem höheren Verhältnis als 1 : 1 eine vermehrte Leberspeicherung aufwiesen. Die Kopplung eines Diamid-dimercaptid-N₂S₂-Liganden an Proteine (A. R. Fritzberg, S. Kasina, J. M. Reno et al., J. Nucl. Med. 27, 957 bis 958, 1986) erfolgt über eine zusätzliche funktionelle Gruppe. So wurde z. B. 4,5-Di(S-ethylcarbonylmercaptoacetamid)-pentanoyl-N-hydroxysuccinimid mit einem anti-Melanomantikörper umgesetzt. Das entstandene Konjugat wurde bei pH 8 und 50°C mit Tc-99m-Tartratlösung inkubiert. Nach einer Stunde waren 78 % des Technetiums vom Tartrat auf den Antikörper übertragen.

Um Technetium-99m diagnostisch breit nutzen zu können, ist es notwendig, dieses Nuklid in das zu untersuchende Organ selektiv zu transportieren. Aus anderen Organen oder Organsystemen sollte das Technetium-99m wieder schnell eliminiert oder überhaupt nicht hingebracht werden, um jede unnötige Strahlenbelastung für den Patienten zu vermeiden. Zu diesem Zweck werden bisher vorwiegend Substanzen eingesetzt, die direkt mit Technetium-99m markierbar sind und eine hohe Organspezifität aufweisen. Darüber hinaus gibt es jedoch eine Reihe von Substanzen, die zwar eine hohe Organspezifität aufweisen, aber nicht direkt markierbar sind. Dies können Proteine (Fibrinogen, Humanserumalbumin), monoclonale Antikörper, Antikörperfragmente, diagnostische Peptide (Amidinophenylalaninderivate; EP 0508220, EP 0513543), Enzyme (Streptokinase, Lactatdehydrodgenase), Zucker (Dextran, Glucose) oder auch Polymere sein. Dazu gehören ebenfalls niedermolekulare Substanzen wie etwa Fettsäuren, die sich durch den hohen Energiebedarf des Herzens im Myocardgewebe anreichern. Um diese Substanzen markieren zu können, werden sie mit Komplexbildnern gekoppelt, die ihrerseits Technetium-99m fest binden können.

Als geeignete Komplexbildner für die Komplexierung von Metallionen sind makrozyklische Amine, u. a. auch Cyclame, bekannt. Die Komplexierungsausbeute liegt für den Technetium-Cyclam Komplex unter geeigneten Bedingungen bei 99 %. Einzelheiten über Technetium-Aminkomplexe sind in D. E. Troutner, J. Simon, A. R. Ketrin, W. A. Volkert, R. A. Holmes, J. Nucl. Med. 21 (1980), 443 oder S. A. Zuckmann, G. M. Freeman, D. E. Troutner, W. A. Volkert, R. A. Holmes, D. G. van der Keer, E. K. Barefiled. Inorg. Ch. 20 (1981), 3386 oder J. Simon, D. Troutner, W. A. Volkert, R. A. Holmes, Radiochem. Radioanal. Lett. 47 (1981), 111 erwähnt. Auch substituierte Cyclame sind, sowohl am 1-Stickstoff, als auch am 6-Kohlenstoff substituiert, bekannt (A. R. Ketrin, D. E. Troutner et al., Int. J. Nucl. Med. Biol. 11 (1984), 113 oder J. Simon. Diss. Abstr. Int. B42 (1981), 645 oder M. Struden, T. A. Kaden, Helv. Chim. Acta 69 (1986), 2081 oder E. Kimura, R. Machida, M. Kodama, J. Am. Chem. Soc. 106 (1984), 5497).

Eine Reihe von Versuchen, Amin- und auch andere Liganden an Proteine zu konjugieren (s. Fritzberg et al., J. Nucl. Med. 27 (1986), 957 oder Tolman et al., J. Nucl. Med. 25 (1984), 20 oder Arano et al., Int. J. Nucl. Med. Biol. 12 (1986) 425) führten zu Produkten, die die hohen in vivo Stabilitätsansprüche nicht oder nur teilweise erfüllten.

Der vorliegenden Erfindung lag daher die Aufgabe zugrunde, geeignete Chelatbildner zur Herstellung äußerst stabiler, insbesondere radioaktiver Technetium- und Rheniumkomplexe zur Verfügung zu stellen, die auf einfache Weise herstellbar und für die Markierung von Substanzen einsetzbar sind.

Die Lösung dieser Aufgabe gelang überraschenderweise mit substituierten Aminodialkylphosphonsäuren der Formel (I) 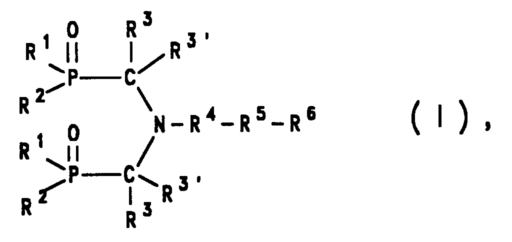
worin
- R¹: Hydroxyl, Amino oder Thiol,
- R²: Wasserstoff, Hydroxyl, Amino, Thiol, unverzweigtes oder verzweigtes C₁- bis C₄-Alkyl, Phenyl oder Benzyl, unverzweigtes oder verzweigtes C₁- bis C₄-Alkyloxy, Phenyloxy oder Benzyloxy, unverzweigtes oder verzweigtes C₁- bis C₄-Alkylamino, Phenylamino oder Benzylamino, unverzweigtes oder verzweigtes C₁- bis C₄-Mercaptoalkyl, Thiophenyl oder Mercaptobenzyl sind,
- R³ und R^{3'}: gleich oder verschieden sind und Wasserstoff oder (C₁-C₄)-Alkyl, sin d,
- R⁴: unverzweigtes oder verzweigtes C₀- bis C₆-Alkylen oder ortho-, meta- oder para-C₇-C₁₅-Aralkylen,
- R⁵: Amino, Hydroxyl, Thiol, eine Ester- oder eine Amidgruppe, -CH₂-, -CH(R⁷)-, -CH(NH₂)- oder -CH(OH)- ist
und
- R⁶: -COOH, sofern R⁵ -CH₂-, -CH(R⁷)-, -CH(NH₂)- oder -CH(OH)- bedeutet, oder eine Gruppe der Formel II oder III, sofern R⁵ Amino, Hydroxy oder Thio bedeutet, oder eine Gruppe der Formel IV oder V, sofern R⁵ Amino bedeutet, 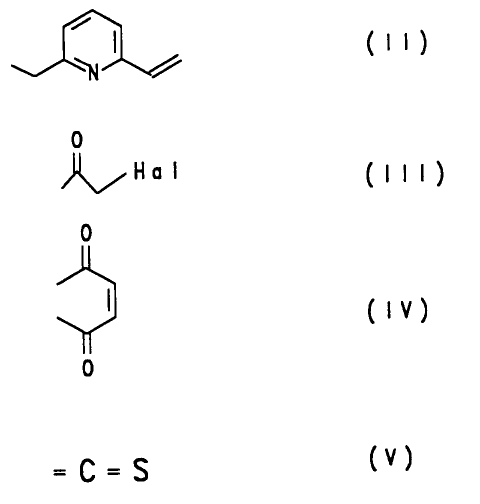 oder -N₃-, -Hal oder -SH ist, wobei Hal für Fluor, Chlor, Brom oder Jod steht und
- R⁷: die Seitenkette einer proteinogenen Aminosäure ist.

Insbesondere betrifft die Erfindung Verbindungen gemäß Formel (I),
worin
- R¹: Hydroxyl, Amino oder Thiol
- R²: Wasserstoff, Hydroxyl, Amino, Thiol, unverzweigtes oder verzweigtes C₁- bis C₄-Alkyl, Phenyl oder Benzyl, unverzweigtes oder verzweigtes C₁- bis C₄-Alkyloxy, Phenyloxy oder Benzyloxy sind,
- R³ und R^{3'}: Wasserstoff oder (C₁-C₄)-Alkyl sind,
- R⁴: unverzweigtes oder verzweigtes C₀- bis C₆-Alkylen oder ortho-, meta- oder para-C₇-C₁₅-Aralkylen ist,
- R⁵: Amino, Hydroxyl, Thiol, eine Ester- oder eine Amidgruppe, -CH₂-, -CH(R⁷)-, -CH(NH₂)- oder -CH(OH)- ist
und
- R⁶: -COOH, sofern R⁵ -CH₂-, -CH(R⁷)-, -CH(NH₂)- oder -CH(OH)- bedeutet, oder eine Gruppe der Formeln II oder III, sofern R⁵ Amino, Hydroxy oder Thio bedeutet, oder eine Gruppe der Formeln IV oder V, sofern R⁵ Amino bedeutet, 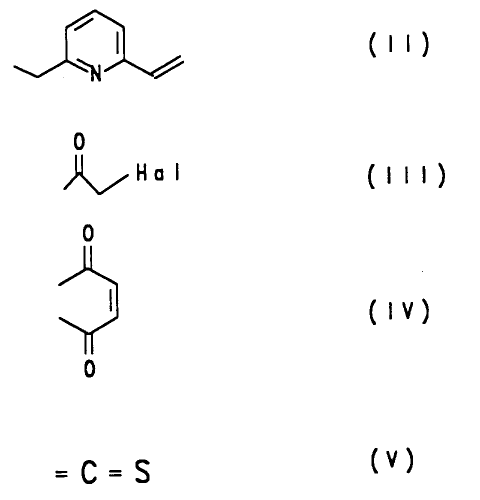 oder -N₃, -Hal oder -SH ist, wobei Hal für Chlor, Brom oder Jod steht und
- R⁷: die Seitenkette einer proteinogenen Aminosäure ist.

Bevorzugt sind Verbindungen gemäß der Formel (I),
worin
- R¹: Hydroxyl, Amino oder Thiol
- R²: Wasserstoff, Hydroxyl, Amino, Thiol, unverzweigtes oder verzweigtes C₁- bis C₃-Alkyl, Phenyl oder Benzyl, unverzweigtes oder verzweigtes C₁- bis C₃-Alkyloxy, Phenyloxy oder Benzyloxy sind,
- R³und R^{3'}: Wasserstoff oder (C₁-C₂)-Alkyl ist,
- R⁴: unverzweigtes oder verzweigtes C₀- bis C₅-Alkylen, ortho-, meta- oder para-C₇-C₁₂-Aralkylen ist,
- R⁵: Amino, Hydroxyl, Thiol, eine Ester- oder eine Amidgruppe, -CH₂-, -CH(R⁷)-, -CH(NH₂)- oder -CH(OH)- ist,
- R⁶: -COOH, sofern R⁵ -CH₂-, -CH(R⁷)-, -CH(NH₂)- oder -CH(OH)- bedeutet, oder eine Gruppe der Formeln II oder III, sofern R⁵ Amino-, Hydroxy- oder Thiogruppe bedeutet, oder eine Gruppe der Formeln IV oder V, sofern R⁵ Amino bedeutet, 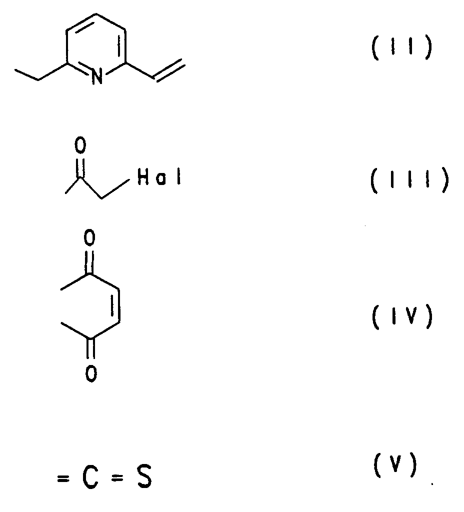 oder Hal bedeutet, wobei Hal für Chlor, Brom oder Jod steht und
- R⁷: die Seitenkette einer proteinogenen Aminosäure ist.

Ganz besonders bevorzugt sind schließlich diejenigen Verbindungen gemäß Formel (I),
worin
- R¹: Hydroxyl, Amino oder Thiol,
- R²: Hydroxyl, Amino oder unverzweigtes oder verzweigtes C₁- bis C₂-Alkyl- sind,
- R³ und R^{3'}: Wasserstoff ist,
- R⁴: unverzweigtes oder verzweigtes C₀- bis C₅-Alkylen, ortho-, meta- oder para-C₇-C₁₂-Aralkylen ist,
- R⁵: Amino, Hydroxyl, eine Ester- oder eine Amidgruppe,-CH₂-, -CH(R⁷)-, -CH(NH₂)- oder -CH(OH)- ist
und
- R⁶: -COOH, sofern R⁵ -CH₂-, -CH(R⁷)-, -CH(NH₂)- oder -CH(OH)- bedeutet oder eine Gruppe der Formeln II oder III, sofern R⁵ Amino- oder Hydroxygruppe bedeutet oder eine Gruppe der Formeln IV, sofern R⁵ Aminogruppe bedeutet, 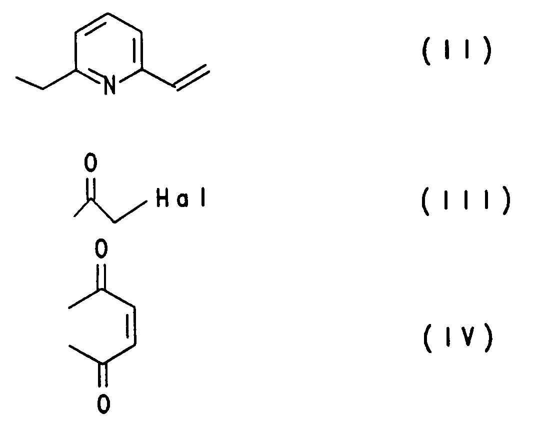 oder Hal, wobei Hal für Chlor, Brom oder Jod steht und
- R⁷: die Seitenkette einer proteinogenen Aminosäure ist.

Unter proteinogener Aminosäure versteht man eine natürliche Aminosäure, insbesondere eine L-Aminosäure.

Die vorliegende Erfindung betrifft weiterhin einen Chelatkomplex, enthaltend eine oder zwei Verbindungen der Formel (I) und mindestens ein Technetium- oder Rheniumion. Bevorzugt sind Chelatkomplexe mit einem Technetium- oder Rheniumion.

Die vorliegende Erfindung betrifft weiterhin ein Konjugat enthaltend eine Verbindung gemäß Formel I und, über die Seitenkette R⁶ gebunden, eine "Substanz von diagnostischem und/oder therapeutischem Interesse". Unter "Substanzen von diagnostischem und/oder therapeutischem Interesse" werden in erster Linie solche Verbindungen verstanden, die in der medizinischen Diagnostik als "Transportsubstanzen" eingesetzt werden können, also meist organspezifische Substanzen, wie Antikörper, Antikörperfragmente [z. B. F(ab')₂- oder Fab'-Fragmente], Proteine (z. B. Fibrinogen), Peptide (z. B. Somatostatin), Oligonukleotide, Zucker (z. B. Dextran, Glucose) oder auch Polymere. Andererseits können aber auch generell solche Substanzen mit den erfindungsgemäßen nichtcyclischen Chelatbildnern bzw. daraus hergestellten Chelatkomplexen markiert werden, die mit deren funktionellen Gruppe an der Seitenkette unter Ausbildung einer chemischen Bindung reagieren. Gedacht ist hierbei z. B. an die "Überwachung" von chemischen Substanzen in Produktionsanlagen, die Bestimmung ihrer Konzentration, Strömungsgeschwindigkeit und Verweilzeit.

Die Herstellung der erfindungsgemäßen Chelatbildner auf der Basis von Aminodialkylphosphoroxiden erfolgt auf zwei unterschiedlichen Wegen.
a) Ausgehend von Verbindungen mit freien terminalen Aminogruppe der Formel VI, z.B. kommerziell erhältlichen Aminosäuren, werden alle vorhandenen funktionellen Gruppen, außer der Aminogruppe, wie literaturbekannt mit Schutzgruppen (Protective Groups in Organic Synthesis, Second Edition, T.W. Greene, P. G. M. Wuts, John Wiley & Sons, Inc., New York, 1991) blockiert. Die dann freie Aminogruppe kann z.B. zur Herstellung der Aminodialkylphosphoroxidderivate durch Reaktion einer Carbonylverbindung der Formel VII und einer Phosphorverbindung der Formel VIII umgesetzt werden. Das Reaktionsprinzip ist in Houben Weyl, Methoden der Organischen Chemie, Georg Thieme Verlag, Stuttgart 1982, E2, S. 130 ff und S. 302 ff beschrieben. Nach Abspaltung der Schutzgruppen entsteht eine Verbindung der Formel I.
   Schema I verdeutlicht die Reaktionsfolge. 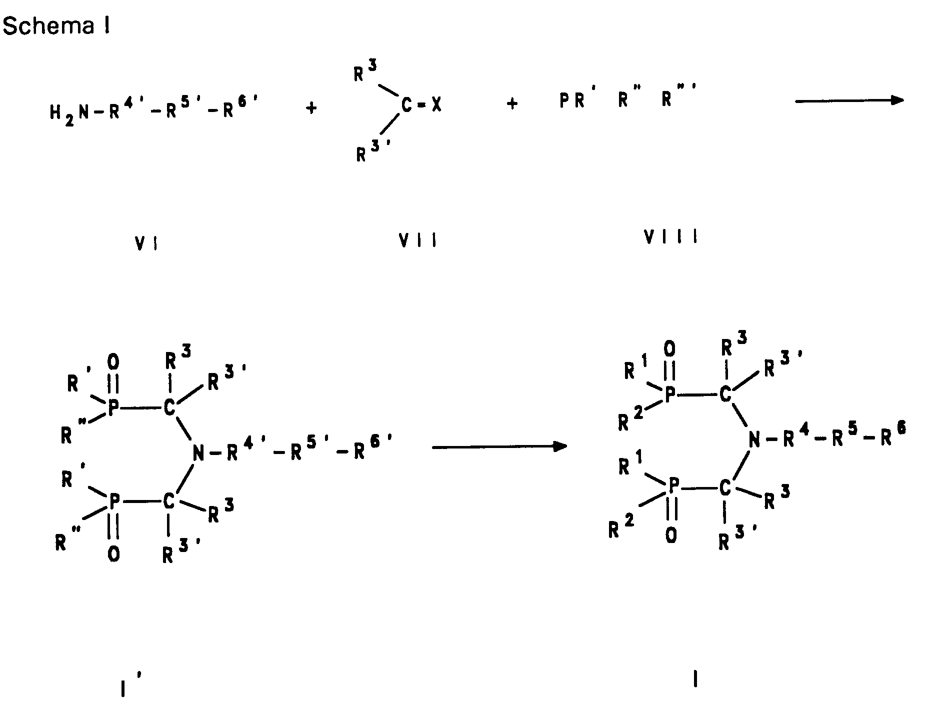 In Schema I bedeuten R^{4'}, R^{5'}, R^{6'} die mit Schutzgruppen versehenen Gruppen R⁴, R⁵, R⁶, sofern R⁴, R⁵ oder R⁶ eine Gruppe bedeutet, die geschützt werden kann und muß. X bedeutet Sauerstoff oder Stickstoff, der substituiert wird durch den Stickstoff bzw. den Phosphor der Verbindungen der Formeln VI bzw. VIII.
   R', R'' und R''' bedeuten Halogen, insbesondere Chlor, Brom, Fluor, Wasserstoff, Hydroxyl, Amino, Thiol, unverzweigtes oder verzweigtes C₁- bis C₄-Alkyl, Phenyl oder Benzyl, unverzweigtes oder verzweigtes C₁- bis C₄-Alkyloxy, Phenyloxy oder Benzyloxy, unverzweigtes oder verzweigtes C₁- bis C₄-Alkylamino, Phenylamino oder Benzylamino, unverzweigtes oder verzweigtes C₁- bis C₄-Mercaptoalkyl, Thiophenyl oder Mercaptobenzyl. Sofern R' und R'' eine der oben genannten Gruppen bedeutet - außer Hydroxyl, Amino oder Thiol - wird bei der Überführung einer Verbindung der Formel I' in eine Verbindung der Formel I R' und R'' entweder verseift, so daß R¹ und R² Hydroxyl bedeuten, oder entsprechend den dem Fachmann bekannten Methoden R' und R'' umgesetzt in R¹ und R², die Amino, Hydroxyl oder Thiol bedeuten. Anschließend werden die Schutzgruppen der Reste R^{4'}, R^{5'} und R^{6'} abgespalten, so daß Gruppen R⁴, R⁵ und R⁶ entstehen.
b) Ausgehend von Benzylamin der Formel IX werden die Aminodialkylphosphoroxdderivate der Formel I durch Reaktion mit einer Carbonylverbindung der Formel VII und einer Phosphorverbindung der Formel VIII hergestellt. Das Reaktionsprinzip ist in "Houben Weyl, Methoden der Organischen Chemie", Georg Thieme Verlag, Stuttgart 1982, S. 130 ff und S. 302 ff beschrieben. Nach Abspaltung der Benzylschutzgruppe z.B. durch Hydrierung, wird die Verbindung mit der freien terminalen Aminogruppe der Formel X durch Alkylierung mit einem Halogenid oder Tosylat oder durch reduktive Aminierung mit einer Carbonylverbindung der Formel XI am R⁴-Terminus der Einheit Y-R⁴-R⁵-R⁶ verknüpft. Bei der reduktiven Aminierung bildet die Carbonylgruppe mit dem Amin über ein Amid ein Imid. Die Carbonylgruppe wird hierbei in eine Methylengruppe überführt. Die Reaktionsprinzipien sind in "J.March, Advanced Organic Chemistry", 4.Edition, John Wiley & Sons, New York 1992 beschrieben. Nach Abspaltung der verbleibenden Schutzgruppen entsteht die Zielverbindung. Schema II verdeutlicht die Reaktionsfolge.

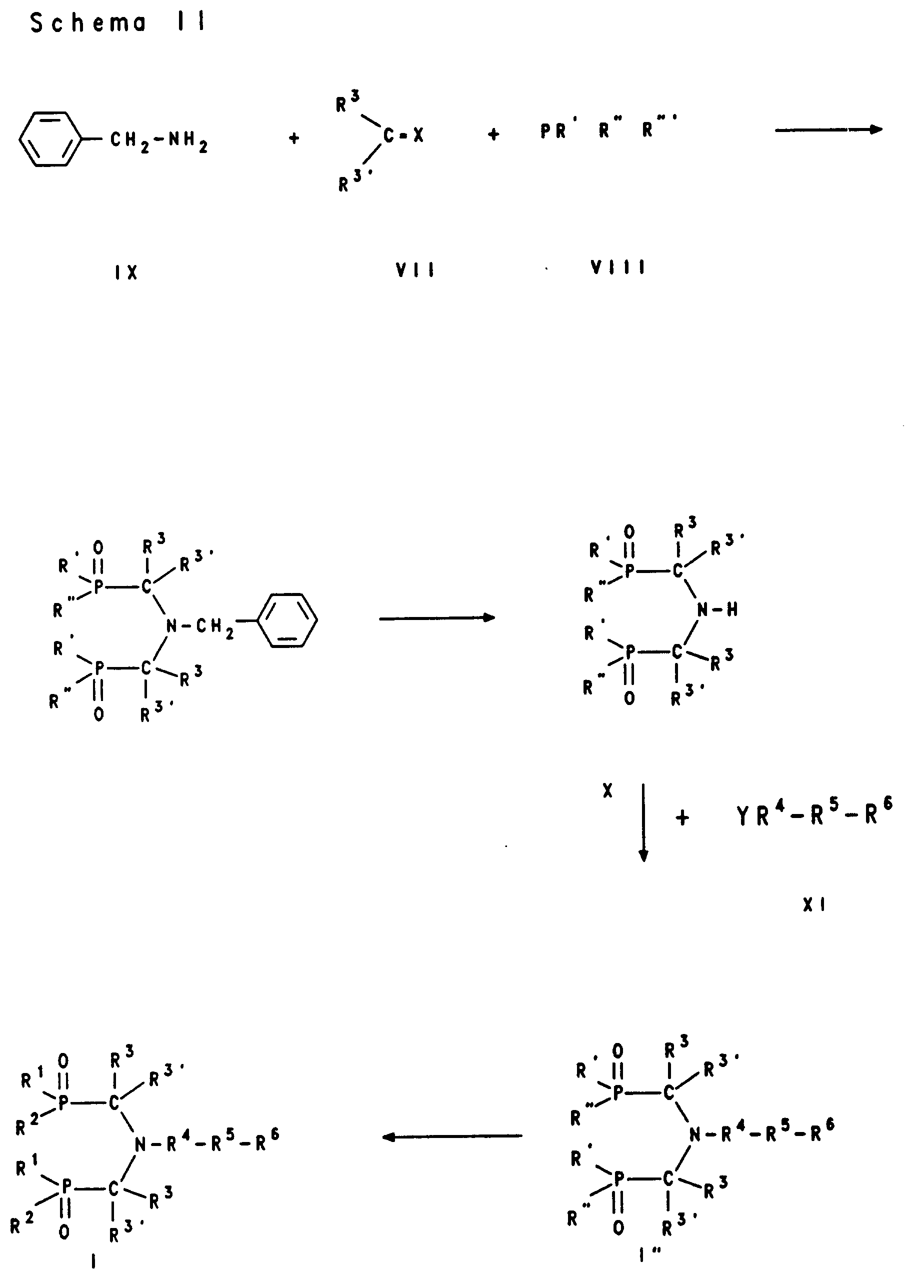

In Schema II haben die Reste R', R'', R''' und R⁴, R⁵ und R⁶ dieselbe Bedeutung wie in Schema I. Y bedeutet hier Halogen, Tosylat oder eine Carbonylgruppe. Auch hier gilt, daß sofern R' und R'' eine der oben genannten Gruppen bedeuten - außer Hydroxyl, Amino oder Thiol - bei der Überführung einer Verbindung der Formel I'' in eine Verbindung der Formel I R'und R'' entweder verseift werden, so daß R¹ und R² Hydroxyl bedeuten, oder entsprechend eine dem Fachmann bekannten Methode R' und R'' in R¹ und R² umgesetzt werden, die Amino, Hydroxyl oder Thiol bedeuten.

Bei dem Verfahren zur Herstellung des Konjugates wird schließlich der nichtcyclische Chelatbildner der Formel I, der am Ende der Seitenkette (R⁶ in Formel (I)) eine funktionelle Gruppe trägt, mit Hilfe dieser funktionellen Gruppe an die zu markierende Substanz (organspezifische Substanz) gebunden. Die zu markierende Substanz trägt hierzu selbst mindestens eine reaktive Gruppe, insbesondere eine Amino, -Carboxy oder Thiolgruppe, so daß unter schonenden bedingungen die Kopplung bzw. die herstellung des Konjugates durchzuführen ist. Gegebenenfalls nach entsprechender Reinigung des Konjugats (z. B. durch Ultrafiltration oder Dialyse bei Proteinen oder Polymeren, durch Säulenchromatographie bei niedermolekularen Substanzen wie Steroiden oder Lipiden) werden Technetium in Form von Pertechnetat bzw. Rhenium in der Form von Perrhenat und ein geeignetes Reduktionsmittel zur Reduktion des Pertechnetats bzw. Perrhenats in die für die Komplexierung benötigte Oxidationsstufe, in beliebiger Reihenfolge oder gemeinsam zugegeben. Das markierte Substrat wird gegebenenfalls nochmals gereinigt. Alternativ kann auch zunächst der Technetium- bzw. Rheniumkomplex des nichtcyclischen Chelatbildner auf Basis von Aminodialkylphosphoroxiden erzeugt werden und dann dieser mit der Substanz zum Konjugat umgesetzt werden. Hierbei verläuft die Komplexierung und Reduktion wie oben beschrieben. Vorzugsweise wird die Komplexierungsreaktion bei basischem pH (4 bis 10) durchgeführt.

Die Reduktion des Pertechnetats bzw. des Perrhenats kann nach literaturbekannten Verfahren erfolgen, bevorzugt mit einer Zinn-II-Verbindung.

Besonders bevorzugt erfolgt die Reduktion mit einem komplexstabilisierten Zinn-II-Salz, nach einem "Markierungsverfahren", wie dies in der deutschen Offenlegungsschrift DE-A 3728599 vorgeschlagen wurde. Dabei wird zunächst die Zinn-II-Verbindung mit einem Komplexbildner, vorzugsweise einer Phosphorverbindung wie einem Phosphonat oder Pyrophosphat oder Citronensäure versetzt, der dafür sorgt, daß die Zinnverbindung vor allem im physiologischen pH-Bereich in Lösung bleibt. Diese komplexstabilisierte Zinn-II-Salz-Lösung kann dann entweder zu der zu markierenden Substanz gegeben werden, worauf die Zugabe der Pertechnetat- bzw. Perrhenat-Lösung erfolgt oder aber zu einer Mischung aus der zu markierenden Substanz und der Pertechnetat- bzw. Perrhenat-Lösung.

Die vorliegende Erfindung betrifft weiterhin ein Diagnostikum enthaltend einen Chelatkomplex, wie oben definiert, mit Technetium-99m und eine organspezifische Substanz entsprechend obiger Definition.

Darüberhinaus betrifft die vorliegende Erfindung ein Therapeutikum enthaltend einen Chelatkomplex entsprechend obiger Definition mit Rhenium-186 oder -188 und eine organspezifische Substanz, wie oben angegeben.

Ferner betrifft die vorliegende Erfindung einen Testkit zum Nachweis von Tumoren, bestehend aus zwei getrennten, lyophilisierten Komponenten, von denen eine einen Antikörper, der gegen Tumor-assoziierte Antigene gerichtet ist, oder dessen F(ab')₂-Fragment mit einem nichtcyclischen Chelatbildner auf Basis von Aminodialkylphosphoroxiden der Formel (I) nach Anspruch 1 verknüpft ist, und die andere ein Reduktionsmittel enthält, das zur Reduktion und Bindung des Technetiums an der Antikörperkomponente benötigt wird.

In einer bevorzugten Ausführungsform wird ein Zinn-II-Salz als Reduktionsmittel verwendet. Besonders bevorzugt wird dabei das Zinn-II-Salz der Methandiphosphonsäure, Aminomethandiphosphonsäure, 3,3-Diphosphonopropionsäure, 3,3-Diphosphono-1,2-propandicarbonsäure, Citronensäure oder der Propan-1,1,3,3-tetraphosphonsäure eingesetzt.

Im folgenden wird die Erfindung anhand von Beispielen näher erläutert.

### 1) Di-[N-methyl-(phosphonyl)]-glycin

### Di-[N-methyl-(diethylphosphonyl)]-glycinmethylester

0,5 g Glycinmethylesterhydrochlorid werden in 100 ml Acetonitril (abs.) suspendiert und nach Zugabe von 3 ml Phosphorigsäurediethylester in Gegenwart von pulverisiertem Molsieb unter Schutzgas zum Rückfluß gebracht. Nach Hinzufügen von 0,9 g Para-Formaldehyd wird bis zur vollständigen Reaktion (ca. 1 h) am Rückfluß erhitzt. Der Niederschlag wird abfiltriert und das Filtrat eingeengt. Der Überschuß an Phosphorigsäuredieethylester wird an der Ölpumpe bei 50°C abdestilliert. Der Rückstand wird an Kieselgel chromatographiert (Eluent: Essigsäureethylester/Methanol 90:10).
Ausbeute: 1,25 g (80%)
¹H-NMR (CDCl₃): 4,1 (POCH₂), 3,3 (PCH₂), 3,85 (NCH₂), 3,7 (-CH₃), 1,3 (POCH₂CH₃) ppm.

### Di-[N-methyl-(phosphonyl)]-glycin

1 g Di-[N-methyl-(diethylphosphonyl)]-glycinmethylester werden in 30 ml konzentrierter Salzsäure ca. 2 h am Rückfluß erhitzt. Nach dem Einengen erhält man 900 mg Rückstand.
Ausbeute: 900 mg (94%).
¹H-NMR (D₂O): 3,3 (PCH₂), 3,95 (NCH₂) ppm.

### 2) ε-N-Di-[N-methyl-(phosphonyl)]-lysin

### ε-N-Di-[N-methyl-(diethylphosphonyl)-α-N-carbonyloxybenzyllysinmethylester

992 g α-N-Carbonyloxybenzyllysinmethylesterhydrochlorid werden in 50 ml Acetonitril (abs.) suspendiert und nach Zugabe von 1,66 g Phosphorigsäurediethylester in Gegenwart von pulverisiertem Molsieb unter Schutzgas zum Rückfluß gebracht. Nach Hinzufügen von 1,2 g Para-Formaldehyd wird bis zur vollständigen Reaktion (ca. 3 h) am Rückfluß erhitzt. Der Niederschlag wird abfiltriert und das Filtrat eingeengt. Der Überschuß an Phosphorigsäuredieethylester wird an der Ölpumpe bei 50°C abdestilliert. Der Rückstand wird an Kieselgel chromatographiert (Eluent: Essigsäureethylester/Methanol 95:5).
Ausbeute: 1,5 g (84%)
¹H-NMR (CDCl₃): 7,4 (Benzyl-H), 5,84 (-NH), 5,1 (Benzyl-CH₂), 4,3 (-CH-), 4,1 (POCH₂), 3,1 (PCH₂), 2,78 (NCH₂), 1,9 - 1,4 (-CH₂-), 1,3 (POCH₂CH₃) ppm.

### ε-N-Di-[N-methyl-(phosphonyl)]-lysin

200 g ε-N-Di-[N-methyl-(diethylphosphonyl)]-α-N-carbonyloxybenzyllysinmethylester werden in 10 ml konzentrierter Salzsäure ca. 6 h am Rückfluß erhitzt. Nach dem Einengen erhält man 140 mg Rückstand.
Ausbeute: 140 mg (97%).
¹H-NMR (D₂O): 3,98 (-CH-), 3,4 (PCH₂), 2,0 - 1,3 (-CH₂-) ppm.

### 3) Synthese von Di-[N-methyl-(diethylphosphonyl)]-glycin

### Di-[N-methyl-(diethylphosphonyl)]-benzylamin

1,07 g Benzylamin werden in 30 ml Acetonitril (abs.) gelöst und nach Zugabe von 5,5 g Phosphorigsäurediethylester in Gegenwart von pulverisiertem Molsieb unter Schutzgas zum Rückfluß gebracht. Nach Hinzufügen von 1,2 g Para-Formaldehyd wird bis zur vollständigen Reaktion (ca. 1 h) am Rückfluß erhitzt. Der Niederschlag wird abfiltriert und das Filtrat eingeengt. Der Überschuß an Phosphorigsäuredieethylester wird an der Ölpumpe bei 50°C abdestilliert. Der Rückstand wird an Kieselgel chromatographiert (Eluent: Essigsäureethylester/Methanol 90:10).
Ausbeute: 1,49 g (37%)
¹H-NMR (CDCl₃): 7,2 (Benzyl-H), 4,1 (POCH₂), 3,95 (Benzyl-CH₂), 3,1 (PCH₂), 1,3 (POCH₂CH₃) ppm.

### Di-[N-methyl-(phosphonyl)]-benzylamin

1 g Di-[N-methyl-(diethylphosphonyl)]-benzylamin werden mit 30 ml konzentrierter Salzsäure ca. 2 h am Rückfluß erhitzt. Nach dem Einengen wird der Rückstand mit 10 ml Methanol verrührt. Der Niederschlag wird abgesaugt, mit Ammoniumhydroxidlösung alkalisch gestellt und eingeengt. Der Rückstand wird an Kieselgel chromatographiert (Eluent: Methanol/Ammoniumhydroxidlösung 90:10).
Ausbeute: 300 mg (34%).
¹H-NMR (D₂O): 7,6 (Benzyl-H), 4,7 (Benzyl-CH₂), 3,55 - 3,35 (PCH₂) ppm.

### Di-[N-methyl-(phosphonyl)]-amin

300 mg Di-[N-methyl-(phosphonyl)]-benzylamin werden in 5 ml Methanol gelöst und mit Palladiumhydroxid auf Aktivkohle in einer Wasserstoffatmosphäre hydriert. Nach 4 Stunden wird der Palladiumkatalysator abfiltriert und die Lösung eingeengt.
Ausbeute: 210 mg (95%)
¹H-NMR (D₂O): 3,61 - 3,40 (PCH₂) ppm.

### Di-[N-methyl-(diethylphosphonyl)]-glycin

210 mg Di-[N-methyl-(diethylphosphonyl)]-amin werden in flüssigem Ammoniak gelöst, mit 234 mg Natriumamid und 416 mg Iodessigsäure-Natriumsalz versetzt. Nach 8 h wird mit Ammoniumchlorid neutralisiert, der Ammoniak verdampft. Der Rückstand wird an Kieselgel chromatographiert (Eluent: Methanol/Ammoniumhydroxidlösung 100:0 - 1:1).
Ausbeute: 193 mg (72%).
¹H-NMR (D₂O): 3,3 (PCH₂), 3,95 (NCH₂) ppm.

## Patentansprüche

1. Substituierte Aminodialkylphosphonsäuren der Formel (I) 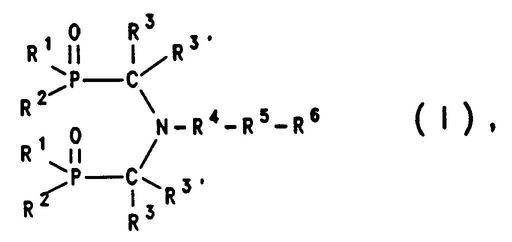 worin
R¹ Hydroxyl, Amino oder Thiol,
R² Wasserstoff, Hydroxyl, Amino, Thiol, unverzweigtes oder verzweigtes C₁- bis C₄-Alkyl, Phenyl oder Benzyl, unverzweigtes oder verzweigtes C₁- bis C₄-Alkyloxy, Phenyloxy oder Benzyloxy, unverzweigtes oder verzweigtes C₁ - bis C₄-Alkylamino, Phenylamino oder Benzylamino, unverzweigtes oder verzweigtes C₁- bis C₄-Mercaptoalkyl, Thiophenyl oder Mercaptobenzyl sind,
R³ und R^{3'} gleich oder verschieden sind und Wasserstoff oder (C₁-C₄)-Alkyl, sind,
R⁴ unverzweigtes oder verzweigtes C₀- bis C₆-Alkylen oder ortho-, meta- oder para-C₇-C₁₅-Aralkylen,
R⁵ Amino, Hydroxyl, Thiol, eine Ester- oder eine Amidgruppe, -CH₂-, -CH(R⁷)-, -CH(NH₂)- oder -CH(OH)- ist
und
R⁶ -COOH, sofern R⁵ -CH₂-, -CH(R⁷)-, -CH(NH₂)- oder -CH(OH)- bedeutet, oder eine Gruppe der Formeln II oder III, sofern R⁵ Amino, Hydroxy oder Thio bedeutet, oder eine Gruppe der Formeln IV oder V, sofern R⁵ Amino bedeutet, 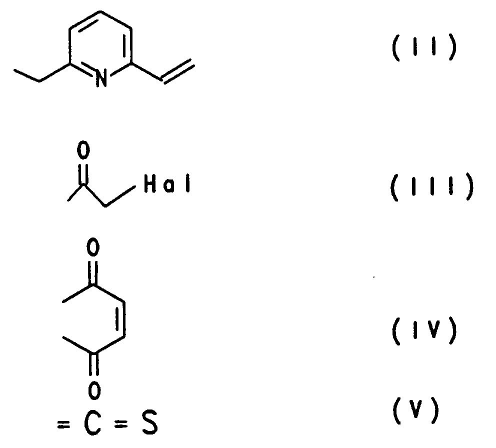 oder -N₃-, -Hal oder -SH ist, wobei Hal für Fluor, Chlor, Brom oder Jod steht und
R⁷ die Seitenkette einer proteinogenen Aminosäure ist.

2. Verbindungen gemäß Formel (I) nach Anspruch 1,
worin
R¹ Hydroxyl, Amino oder Thiol
R² Wasserstoff, Hydroxyl, Amino, Thiol, unverzweigtes oder verzweigtes C₁- bis C₄-Alkyl, Phenyl oder Benzyl, unverzweigtes oder verzweigtes C₁- bis C₄-Alkyloxy, Phenyloxy oder Benzyloxy sind,
R³ und R^{3'} Wasserstoff oder (C₁-C₄)-Alkyl ist,
R⁴ unverzweigtes oder verzweigtes C₀- bis C₆-Alkylen oder ortho-, meta- oder para-C₇-C₁₅-Aralkylen ist,
R⁵ Amino, Hydroxyl, Thiol, eine Ester- oder eine Amidgruppe, -CH₂-, -CH(R⁷)-, -CH(NH₂)- oder -CH(OH)- ist
und
R⁶ -COOH, sofern R⁵ -CH₂-, -CH(R⁷)-, -CH(NH₂)- oder -CH(OH)- bedeutet, oder eine Gruppe der Formeln II oder III, sofern R⁵ Hydroxy oder Thio bedeutet, oder eine Gruppe der Formeln IV oder V, sofern R⁵ Amino bedeutet, 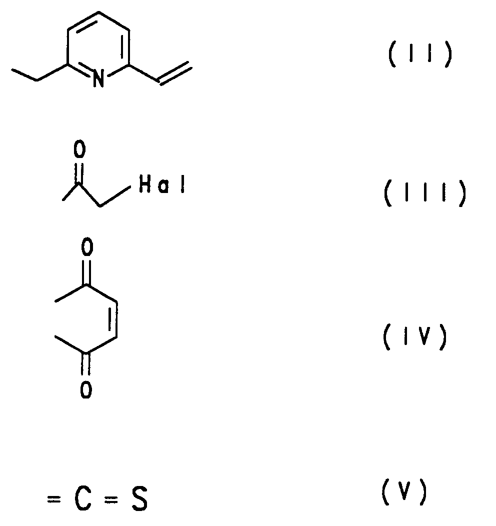 oder -N₃, -Hal oder -SH ist, wobei Hal für Chlor, Brom oder Jod steht und
R⁷ die Seitenkette einer proteinogenen Aminosäure ist.

3. Verbindungen gemäß Formel (I) nach dem Anspruch 1 oder 2,
worin
R¹ Hydroxyl, Amino oder Thiol
R² Wasserstoff, Hydroxyl, Amino, Thiol, unverzweigtes oder verzweigtes C₁- bis C₃-Alkyl, Phenyl oder Benzyl, unverzweigtes oder verzweigtes C₁- bis C₃-Alkyloxy, Phenyloxy oder Benzyloxy sind,
R³und R^{3'} Wasserstoff oder (C₁-C₂)-Alkyl ist,
R⁴ unverzweigtes oder verzweigtes C₀- bis C₅-Alkylen, ortho-, meta- oder para-C₇-C₁₂-Aralkylen ist,
R⁵ Amino, Hydroxyl, Thiol, eine Ester- oder eine Amidgruppe, -CH₂-, -CH(R⁷)-, -CH(NH₂)- oder -CH(OH)- ist,
R⁶ -COOH, sofern R⁵ -CH₂-, -CH(R⁷)-, -CH(NH₂)- oder -CH(OH)- bedeutet, oder eine Gruppe der Formeln II oder III, sofern R⁵ Amino-, Hydroxy- oder Thiogruppe bedeutet, oder eine Gruppe der Formeln IV oder V, sofern R⁵ Amino bedeutet, 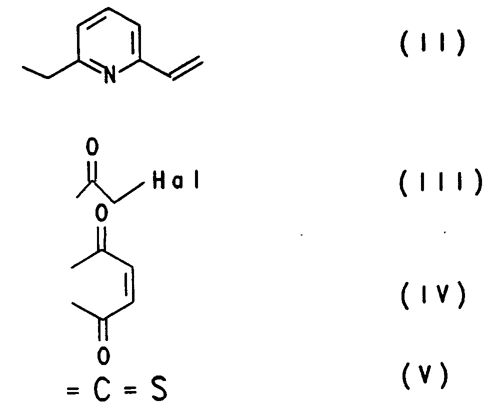 oder Hal bedeutet, wobei Hal für Chlor, Brom oder Jod steht und
R⁷ die Seitenkette einer Aminosäure ist.

4. Verbindungen gemäß Formel (I) nach den Ansprüchen 1 bis 3,
worin
R¹ Hydroxyl, Amino oder Thiol,
R² Hydroxyl, Amino oder unverzweigtes oder verzweigtes C₁- bis C₂-Alkyl- sind,
R³ und R^{3'} Wasserstoff ist,
R⁴ unverzweigtes oder verzweigtes C₀- bis C₅-Alkylen, ortho-, meta- oder para-C₇-C₁₂-Aralkylen ist,
R⁵ Amino, Hydroxyl, eine Ester- oder eine Amidgruppe,-CH₂-, -CH(R⁷)-, -CH(NH₂)- oder -CH(OH)- ist
und
R⁶ -COOH, sofern R⁵ -CH₂-, -CH(R⁷)-, -CH(NH₂)- oder -CH(OH)- bedeutet oder eine Gruppe der Formeln II oder III, sofern R⁵ Amino- oder Hydroxygruppe bedeutet oder eine Gruppe der Formeln IV, sofern R⁵ Aminogruppe bedeutet, 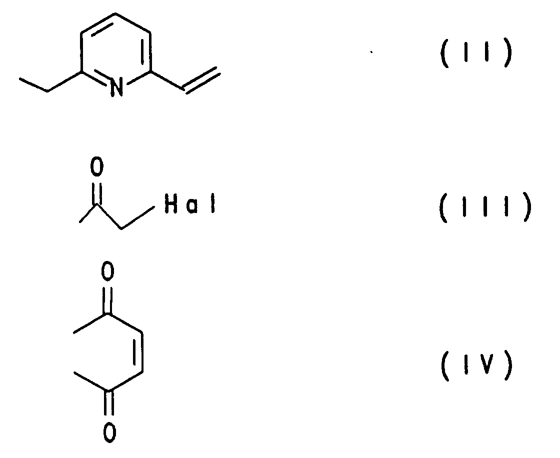 oder Hal, wobei Hal für Chlor, Brom oder Jod steht und
R⁷ die Seitenkette einer proteinogenen Aminosäure ist.

5. Chelatkomplex, enthaltend eine oder zwei Verbindungen der Formel I gemäß den Ansprüchen 1 bis 4 und mindestens ein Technetium- oder Rheniumion.

6. Konjugat, enthaltend einen Chelatkomplex gemäß Anspruch 5 und eine organspezifische Substanz, die über R⁶ einer Verbindung gemäß Formel I gebunden ist.

7. Verfahren zur Herstellung einer Verbindung der allgemeinen Formel I nach den Ansprüchen 1 bis 4, dadurch gekennzeichnet, daß Verbindungen der Formeln VI, VII und VIII zu einer Verbindung der Formel I' umgesetzt werden, 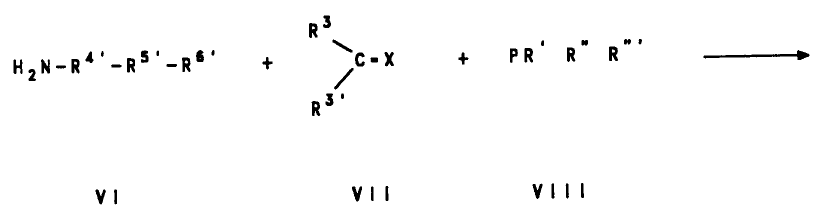 und diese wiederum unter Abspaltung der Schutzgruppen an R^{4'}, R^{5'} und R^{6'} in eine Verbindung der Formel I übergeführt wird, 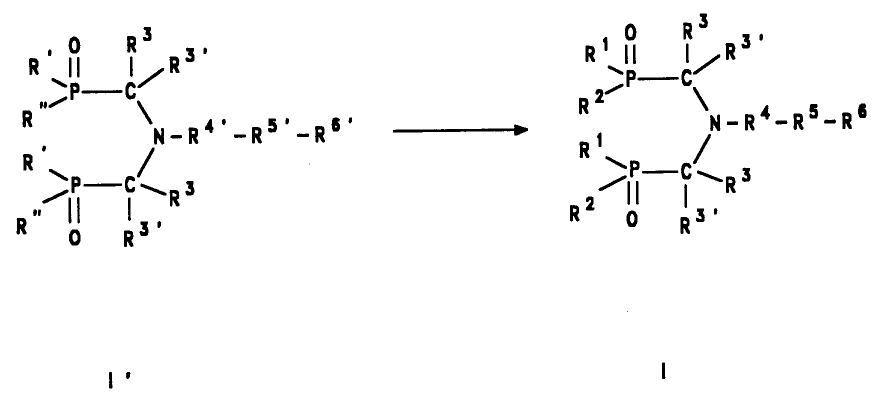 wobei
R^{4'}, R^{5'} und R^{6'} die mit Schutzgruppen versehenen Gruppen R⁴, R⁵ und R⁶,
X Sauerstoff oder Stickstoff und
R', R'' und R''' Halogen, insbesondere Chlor, Brom, Fluor oder ein Rest, der für R² gilt, bedeutet.

8. Verfahren zur Herstellung einer Verbindung der allgemeinen Formel I nach den Ansprüchen 1 bis 4, dadurch gekennzeichnet, daß Verbindungen der Formeln IX, VII und VIII unter Abspaltung der Benzylschutzgruppe in eine Verbindung der Formel X umgesetzt werden, 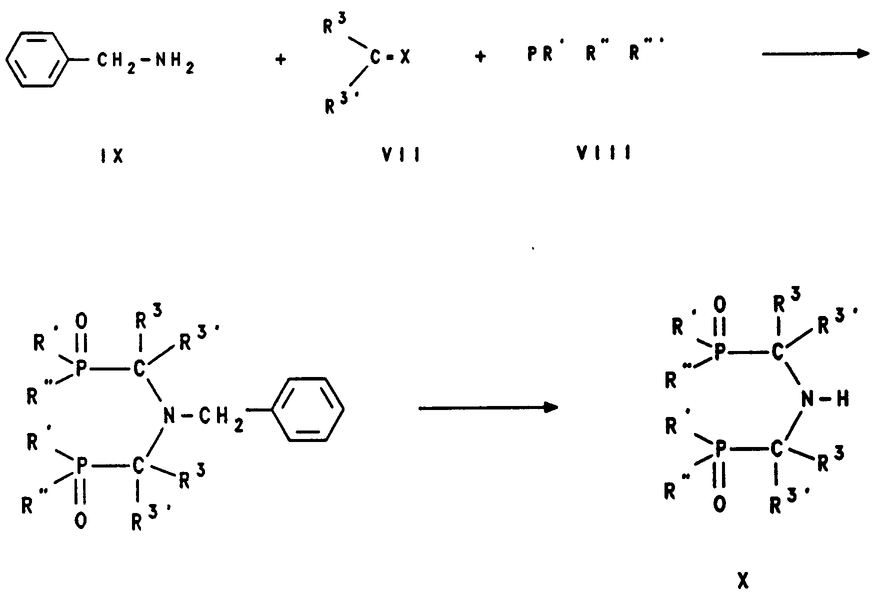 und diese mit Y-R⁴-R⁵-R⁶ (XI) über eine Verbindung der Formel I'' in eine Verbindung der Formel I übergeführt wird, 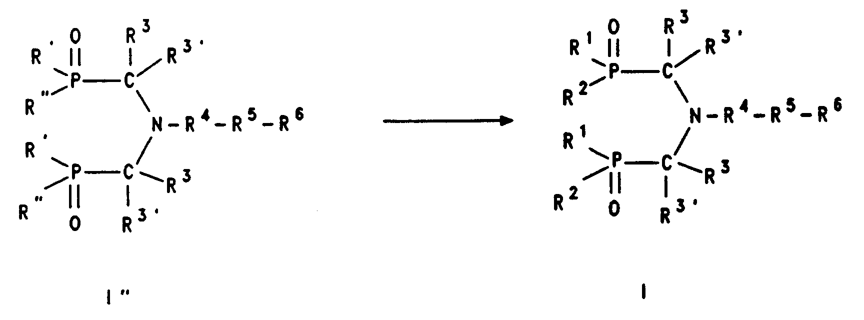 wobei
R', R'', R''' und R⁴, R⁵ und R⁶ wie in Anspruch 7 definiert gelten und
Y Halogen oder Tosylat bedeutet, sowie eine Carbonylgruppe, die nach Kopplung mit dem Amin über das gebildete Imin in eine Methylengruppe übergeführt wird.

9. Diagnostikum enthaltend eine Verbindung gemäß Anspruch 5 und eine organspezifische Substanz.

10. Therapeutikum enthaltend eine Verbindung gemäß Anspruch 5 und eine organspezifische Substanz.

11. Testkit zum Nachweis von Tumoren, enthaltend räumlich getrennt voneinander eine an eine organspezifische Substanz gebundene Verbindung gemäß Anspruch 4 und ein Reduktionsmittel, das zur Reduktion des Technetiums vom Pertechnetat in Technetium (II) befähigt ist.
